# EUROPEAN PATENT APPLICATION

(11) **EP 1 133 996 A1**
(43) Date of publication of application: **19.09.2001**
(21) Application number: 99972548.4
(22) Date of filing: 16.11.1999
(51) Int. Cl.: A61K 45/00, A61K 31/381, A61P 15/00

(54) **MEDICINAL COMPOSITIONS FOR PERIODIC ADMINISTRATION**

(30) Priority: 26.11.1998 JP 33543598; 26.11.1998 JP 33601998
(71) Applicant: TEIKOKU HORMONE MFG. CO., LTD., Tokyo 107-8522 (JP)
(72) Inventor: TAKAHASHI, Hiroo, Sagamihara-shi, Kanagawa 229-0026 (JP); FUJII, Tomohito, Kawasaki-shi Kanagawa 211-0053 (JP); SHIMIZU, Yasushi, Kawasaki-shi Kanagawa 215-0005 (JP); SATO, Kodo, Kashiwa-shi, Chiba 277-0033 (JP)
(74) Representative: Albrecht, Thomas, Dr.
(86) International application number: JP9906383
(87) International publication number: WO0030684

(57) **Abstract**

When it is intended to administer an estrogen antagonist for the treatment or prevention of endometriosis and/or abnormal menstruation, it is unnecessary to administer the drug constantly and continuously throughout the period of time requiring drug administration for therapeutic or preventive purposes (i.e., chronic administration), but therapeutic effects almost comparable to those of chronic administration can be achieved by adminstering the drug for only 5 to 13 consecutive days during a period of time extending from the menstrual period to immediately before ovulation in the menstrual cycle (i.e., periodic administration). Moreover, the periodic administration is substantially free from such side effects as are observed in the chronic administration of estrogen antagonists. Thus, the periodic administration is a useful method for administering an estrogen antagonist for the treatment or prevention of endometriosis and/or abnormal menstruation..

## Description

### Technical Field

This invention relates to pharmaceutical compositions for the treatment or prevention of endometriosis and/or abnormal menstruation which contain an estrogen antagonist as an active ingredient. More particularly, it relates to a pharmaceutical composition for the treatment or prevention of endometriosis and/or abnormal menstruation which contains an estrogen antagonist as an active ingredient, the pharmaceutical composition being characterized in that it is administered for only 5 to 13 consecutive days during a period of time extending from the menstrual period to immediately before ovulation in the menstrual cycle; a method for the treatment or prevention of endometriosis and/or abnormal menstruation which comprises administering such a pharmaceutical composition; the use of an estrogen antagonist in the preparation of such a pharmaceutical composition; and a pharmaceutical product including a packaging material and such an estrogen antagonist-containing pharmaceutical composition within the packaging material.

### Background Art

Endometriosis is a disease caused by the ectopic occurrence of endometrial tissue, usually in various tissues within the pelvis, such as the body of the uterus, ovaries, pelvic peritoneum (in particular, Douglas' cul-de-sac), uterine tubes, uterosacral ligaments, and the rectum. According to the site of the focus, endometriosis is classified into internal endometriosis (adenomyosis uteri) occurring in the myometrium, and external endometriosis occurring at other sites. Endometriosis frequently occur in sexually mature women, and causes recurrent proliferation, bleeding and regeneration ectopically. Consequently, various disorders are produced to result in complaints such as dysmenorrhea, lower abdominal pain, dyspareunia and infertility. For therapeutic purposes, surgical treatment and drug treatment are suitably employed according to symptoms, with consideration for the chief complaint, the presence or absence of childbirth, the patient's age, and the like. With the recent improvement of appliances, more weight is being given to surgical treatment. However, the need for drug treatment is still great because there is a possibility of recurrence even after a conservative operation, not a few cases require drug treatment before and after an operation, and young patients are increasing.

With regard to drug treatment, LH-RH agonist therapy and danazol therapy prevail at present. However, since these drugs have the disadvantage that they relatively often produce side effects such as menopausal syndrome, hepatic dysfunction and defemination, it is desired to develop a drug substantially free of such side effects. On the other hand, since endometriosis involves the proliferation and retrogression of focal tissue repeated in response of the menstrual cycle, and since endometriosis does not develop during pregnancy or after menopause, it is considered to be an estrogen-dependent disease. In recent years, therefore, attempts are being made to apply estrogen antagonists to the treatment of endometriosis. For example, it has been reported that, when tamoxifen, an estrogen antagonist, was administered to patients with endometriosis for 3 to 6 menstrual cycles in which the administration was started on the 5th day of a menstrual cycle and continued except for menstrual periods, subjective symptoms such as lower abdominal pain and lumbago were ameliorated (Journal of the Society for the Research of Endometriosis, Vol. 10, pp. 224-230, 1989). Moreover, a pharmaceutical composition for the suppression of endometriosis which contains raloxifene, an estrogen antagonist, has also been known (EP-A-652,005). However, the chronic administration of drugs for a long period of time generally tends to produce various side effects and hence impose limits on the intended treatment of the patient, so that a satisfactory therapeutic effect may not be achieved. Accordingly, it is desired to use such drugs in a way which can minimize these side effects.

Meanwhile, normal menstruation in mature women is usually believed to be characterized by periodicity occurring in cycles of about 25 to 35 days, a duration of menstruation of about 3 to 7 days, and an amount of menstrual flow of about 50 to 250 g. When the menstrual cycle, the duration of menstruation, or the amount of menstrual flow deviates significantly from the aforesaid normal range, this condition is usually referred to as abnormal menstruation. Dysmenorrhea is a syndrome in which menorrhalgia (e.g., lower abdominal pain and lumbago), nausea, vomiting, headaches and the like develop during or immediately before menstruation, to such an undue extent as to interfere with everyday life and hence require treatment. Dysmenorrhea is classified into primary dysmenorrhea caused by uterine hypoplasia, endocrine ataxia and the like, and secondary dysmenorrhea caused by organic diseases such as endometriosis and hysteromyoma. In its broad sense, abnormal menstruation is said to include dysmenorrhea. Many cases of abnormal menstruation either have no well-defined cause or have intricately tangled causes, and nosotropic therapy or hormonotherapy is generally employed for therapeutic purposes.

As an example of hormonotherapy, a mixed preparation of progestogen and estrogen is usually administered. However, these drugs have problems in that they cannot be used for patients suspected of having estrogen-dependent tumors or thrombosis. Accordingly, it is desired to develop a drug free of these problems.

In recent years, attempts are being made to use estrogen antagonists for the treatment of abnormal menstruation. For example, a pharmaceutical composition for the mitigation of symptoms associated with menstruation which contains raloxifene, an estrogen antagonist, has been reported (EP-A-659,421). Moreover, it has also been reported that raloxifene can be used for the suppression of functional metrorrhagia including hypermenorrhea (EP-A-659,417).

However, the chronic administration of drugs for a long period of time generally tends to produce various side effects and hence impose limits on the intended treatment of the patient, so that a satisfactory therapeutic effect may not be achieved. Moreover, the chronic administration of estrogen antagonists affect the menstrual cycle and generally has the side effect of extending the menstrual cycle. Accordingly, it is desired to use such drugs in a way which can minimize these side effects.

An object of the present invention is to provide a pharmaceutical composition for the treatment or prevention of endometriosis and/or abnormal menstruation which has been designed so as to minimize the side effects of estrogen antagonists.

Another object of the present invention is to provide the use of an estrogen antagonist in the preparation of such a pharmaceutical composition, and a pharmaceutical product comprising a packaging material and such a pharmaceutical composition within the packaging material.

### Disclosure of the Invention

The present inventors carried out various investigations on pharmaceutical composition for the treatment or prevention of endometriosis and/or abnormal menstruation which can minimize the side effects of estrogen antagonists. As a result, it has now been found that, when it is intended to administer an estrogen antagonist for the treatment and/or prevention of endometriosis and/or abnormal menstruation, it is unnecessary to administer the drug constantly and continuously throughout the period of time requiring drug administration for therapeutic or preventive purposes (i.e., chronic administration), but it will be sufficient to administer the drug for only 5 to 13 consecutive days during a period of time extending from the menstrual period to immediately before ovulation in the menstrual cycle. Thus, therapeutic effects almost comparable to those of chronic administration can be achieved by administering the drug for only such 5 to 13 consecutive days in each menstrual cycle. Consequently, the periodic administration can produce an excellent effect in that the dosage of the estrogen antagonist can be kept on a considerably lower level as compared with chronic administration and, therefore, the periodic administration is substantially free from such side effects as are observed in the chronic administration of estrogen antagonists. The present invention has been completed on the basis of this finding.

Thus, according to the present invention, there is provided a pharmaceutical composition for the treatment or prevention of endometriosis and/or abnormal menstruation which contains an estrogen antagonist as an active ingredient, the pharmaceutical composition being characterized in that it is administered for only 5 to 13 consecutive days during a period of time extending from the menstrual period to immediately before ovulation in the menstrual cycle.

Moreover, according to the present invention, there is provided an agent for the treatment or prevention of endometriosis and/or abnormal menstruation which contains an estrogen antagonist as an active ingredient, the agent being administered for only 5 to 13 consecutive days during a period of time extending from the menstrual period to immediately before ovulation in the menstrual cycle.

Furthermore, according to the present invention, there is provided a method for the treatment or prevention of endometriosis and/or abnormal menstruation which comprises administering an estrogen antagonist for only 5 to 13 consecutive days during a period of time extending from the menstrual period to immediately before ovulation in the menstrual cycle.

The present invention also provides the use of an estrogen antagonist in the preparation of a pharmaceutical composition for the treatment or prevention of endometriosis and/or abnormal menstruation which is administered for only 5 to 13 consecutive days during a period of time extending from the menstrual period to immediately before ovulation in the menstrual cycle.

Still further, according to the present invention, there is provided a pharmaceutical product for the treatment or prevention of endometriosis which includes a packaging material and an estrogen antagonist-containing pharmaceutical preparation within the packaging material, the pharmaceutical product being characterized by having, on or within the packaging material, an indication or document (i.e., directions for use) showing the instruction that the estrogen antagonist-containing pharmaceutical preparation should be administered for only 5 to 13 consecutive days during a period of time extending from the menstrual period to immediately before ovulation in the menstrual cycle.

In this description, the term "endometriosis" is used to include adenomyosis uteri observed in the myometrium, too. The term "abnormal menstruation" is used to comprehend dysmenorrhea, abnormalities in the amount of menstruation (e.g., hypermenorrhea, hypomenorrhea, etc.), and abnormalities in the menstrual cycle or the duration of menstruation (e.g., oligomenorrhea, polymenorrhea, paramenia, etc.).

As used herein, the term "estrogen antagonist" means any compound that has a strong affinity for estrogen receptors and hence functions to block the action of estrogens. Specific examples thereof include tamoxifen (THE MERCK INDEX 12th EDITION No. 9216), raloxifene (THE MERCK INDEX 12th EDITION No. 8281), clomiphene (THE MERCK INDEX 12th EDITION No. 2446), toremifene (THE MERCK INDEX 12th EDITION No. 9688), droloxifene (THE MERCK INDEX 12th EDITION No. 3502), and the compounds described in the literature including, for example, EP-A-641, 791, Japanese Patent Laid-Open No. 53851/'77, Japanese Patent Laid-Open No. 57277/'93, Japanese Patent Laid-Open No. 112510/'93, Japanese Patent Laid-Open No. 49673/'94, Japanese Patent Laid-Open No. 321937/'94, Japanese Patent Laid-Open No. 333366/'96, Published Japanese Translation of PCT International Publication No. 503215/'98, Japanese Patent Laid-Open No. 130211/'98, Japanese Patent Laid-Open No. 130212/'98, and Japanese Patent Laid-Open No. 130261/'98. Among these estrogen antagonists, the especially preferred compounds include tamoxifen, raloxifene, clomiphene, toremifene, droloxifene and (6-hydroxy-2-cyclohexylbenzo[b]thien-3-yl) [4-[2-(1-piperidinyl)ethoxy]phenyl]methanone (hereinafter referred to as "TZE-5323").

If necessary, the estrogen antagonist may be reacted with an inorganic or organic acid or an inorganic or organic base to form a pharmaceutically acceptable salt. The inorganic acids which can be used to form a salt include, for example, hydrochloric acid, hydrobromic acid, nitric acid, sulfuric acid and phosphoric acid, and the organic acids which can be used include, for example, acetic acid, propionic acid, oxalic acid, malonic acid, succinic acid, lactic acid, malic acid, tartaric acid, citric acid, benzoic acid and methanesulfonic acid. On the other hand, the inorganic bases which can be used to form a salt include, for example, sodium hydroxide, ammonium hydroxide, calcium carbonate, sodium hydrogen carbonate and calcium hydroxide, and the organic bases which can be used include, for example, methylamine, diethylamine, dicyclohexylamine, ethanolamine and morpholine.

According to an investigation conducted by the present inventors, it will be sufficient to administer the estrogen antagonist for only 5 to 13 consecutive days during a period of time extending from the menstrual period to immediately before ovulation in the menstrual cycle. The period of administration is preferably selected from 6 to 11 consecutive days, and more preferably from 7 to 9 consecutive days. The time at which the administration of the estrogen antagonist is started generally lies between the 1st and the 7th day after the onset of menstruation in each menstrual cycle, and it is especially preferable to start the administration on any of the 3rd to 5th days. On the other hand, the time at which the administration is terminated generally lies between the 7th and the 14th day after the onset of menstruation in each menstrual cycle, and it is especially preferable to terminate the administration on any of the 9th to 11th days.

The pharmaceutical composition for the treatment or prevention of endometriosis and/or abnormal menstruation in accordance with the present invention may be used in the form of a pharmaceutical preparation having any of dosage forms including solid forms (e.g., tablets, hard capsules, soft capsules, granules, powders, fine subtilaes, pills and troches), semisolid forms (e.g., suppositories and ointments), and liquid forms (e.g., injections, emulsions, suspensions, elixirs, lotions and sprays). The additives which can be used to make such pharmaceutical preparations include, for example, starch, glucose, sucrose, lactose, fructose, maltose, mannitol, sorbitol, cyclodextrin, silicic acid derivatives, methylcellulose, carboxymethylcellulose and its salts, alginates, gelatin, polyvinyl pyrrolidone, calcium carbonate, sodium hydrogen carbonate, magnesium carbonate, talc, magnesium stearate, gum arabic, polyethylene glycol, alkyl p-hydroxybenzoates, cetyl alcohol, syrups, ethanol, propylene glycol, vaseline, carbowax, glycerol, sodium chloride, sodium sulfite, sodium phosphate and citric acid.

If necessary, the pharmaceutical composition of the present invention may also be used in the form of a sustained release preparation. Such a sustained release preparation may be made according to a per se known method, such as a method comprising coating the preparation with a material selected from fats and oils (e.g., triglycerides), fatty acid esters of polyglycerol, hydroxypropyl cellulose and the like.

The content of the estrogen antagonist in the pharmaceutical preparation may vary according to its dosage form and the like. However, it is generally desirable that the pharmaceutical preparation contains the estrogen antagonist at a concentration of 0.1 to 50% by weight in the case of solid and semisolid forms, and at a concentration of 0.05 to 10% by weight in the case of liquid forms.

The dosage of the estrogen antagonist administered for the treatment or prevention of endometriosis and/or abnormal menstruation according to the present invention may vary widely according to the route of administration, the type and severity of symptoms, the doctor's diagnosis, and the like. However, the generally acceptable effective daily dose is in the range of about 0.1 to about 1,000 mg per day and preferably about 30 to about 600 mg per day. Nevertheless, it is a matter of course that, depending on the type and severity of symptoms and the doctor's diagnosis as described above, the estrogen antagonist may be administered in a dose less than the lower limit of the aforesaid range or greater than the upper limit thereof The aforesaid dose may be administered once a day or in several divided doses.

### Examples

The present invention is more specifically explained with reference to the following tests and examples.

In the following tests, TZE-5323 was used as the estrogen antagonist.

### Test 1

Among female Cynomolgus monkeys showing normal menstrual cycles, animals exhibiting a high serum CA125 level and hence suspected of spontaneous endometriosis were laparotomized in conformity with the menstrual cycle, and the individuals in which the presence of a focus was confirmed were divided into three groups according to the serum CA125 level. After the individuals recovered from diagnostic laparotomy, predetermined blood markers (estradiol, progesterone, LH, FSH, etc.) were determined by using the first menstrual cycle as a control cycle. Starting from the second menstrual cycle, test compound was administered in the following manner. TZE-5323 was diluted with fructose to form a 25-fold triturated powder, and the placebo comprised fructose alone. They were sprinkled over apple slices and administered orally.

| Test compound | Dose (mg/kg) | Period of administration |
|---|---|---|
| Placebo (fructose alone) | 0 | 3rd to 9th days after the onset of menstruation in each of the 2nd to 4th menstrual cycles |
| TZE-5323 (periodic administration group) | 3 | 3rd to 9th days after the onset of menstruation in each of the 2nd to 4th menstrual cycles |
| TZE-5323 (chronic administration group) | 3 | Continuous administration for 90 days beginning on the 3rd day after the onset of menstruation in the 2nd menstrual cycle |

After completion of the test, all animals of each group were put to death by exsanguination under anesthesia, and subjected to histopathologic examinations of the ovaries, the uterus and the focus of endometriosis.

The above-described test revealed that the administration of TZE-5323 was more effective in the treatment of endometriosis as compared with the administration of a placebo. Both the group having undergone the periodic administration of TZE-5323 and the group having undergone the chronic administration of TZE-5323 showed an ameliorating effect on endometriosis. However, the chronic administration group showed side effects such as a disturbance of the menstrual cycle, a rise in basal blood LH level, the disappearance of an LH surge, and the ensuing steady increase in blood estradiol level, whereas the periodic administration group did not shown such side effects as were observed in the chronic administration group.

### Test 2

Cynomolgus monkeys showing normal menstrual cycles were randomly divided into three groups. Starting on the 3rd day after the onset of menstruation (counted by regarding the day of the onset of menstruation as day 0), test compound was administered in the following manner. TZE-5323 was used in the form of a suspension in a 2% aqueous solution of Tween 80, and the placebo comprised a 2% aqueous solution of Tween 80 alone. They were forcedly administered into the stomach through a nasogastric tube.

| Test compound | Dose (mg/kg) | Period of administration |
|---|---|---|
| Placebo (2% aqueous solution of Tween 80 alone) | 0 | 3rd to 9th days after the onset of menstruation in each of the 1st to 3rd menstrual cycles |
| TZE-5323 (periodic administration group) | 3 | 3rd to 9th days after the onset of menstruation in each of the 1st to 3rd menstrual cycles |
| TZE-5323 (chronic administration group) | 3 | 3rd day after the onset of the 1st menstrual cycle to 2nd day after the onset of the 4th menstrual cycle, or (in cases where no menstruation was observed after the start of the administration) continuous administration for 90 days |

During the period of administration, the general condition of the animals was observed every day. Moreover, blood samples were collected on the 2nd, 7th, 10th, 13th, 16th, 20th, 24th and 28th days after the onset of menstruation in each menstrual cycle (in cases where the next menstruation was not observed 5 days after blood sample collection on the 28th day, blood samples were collected on every 5th day), and blood markers (estradiol, progesterone, LH and FSH) were determined.

After completion of the test, all animals of each group were put to death by exsanguination under anesthesia, and subjected to histopathologic examinations of various organs and tissues.

In the above-described test, the group having undergone the chronic administration of TZE-5323 showed side effects such as a disturbance of the menstrual cycle, a rise in basal blood LH level, the disappearance of an LH surge, and the ensuing steady increase in blood estradiol level. On the other hand, the periodic administration group did not show such side effects as were observed in the chronic administration group. Moreover, the menstrual cycle was extended by about 3 to 5 days, and the extended menstrual cycle was repeated within an error of about 1 day. The length of the menstrual cycle in the placebo administration group showed a greater variation as compared with the periodic administration group.

### Example 1

| Tablets | |
|---|---|
| mg/tablet | |
| TZE-5323 | 25.0 |
| Starch | 5.0 |
| Lactose | 107.0 |
| Carboxymethylcellulose calcium | 10.0 |
| Talc | 1.0 |
| Magnesium stearate | 2.0 |
| | 150.0 |

TZE-5323 was reduced to a particle size of 70 µm or less. Then, starch, lactose and carboxymethylcellulose calcium were added thereto and thoroughly mixed therewith. After the addition of 10% starch paste, the above powder mixture was agitated and blended to prepare granules. After drying, these granules were adjusted to a particle diameter of about 1,000 µm, and mixed with talc and magnesium stearate. The resulting mixture was formed into tablets.

### Example 2

| Tablets | |
|---|---|
| mg/tablet | |
| TZE-5323 | 50.0 |
| Starch | 5.0 |
| Lactose | 132.0 |
| Carboxymethylcellulose calcium | 10.0 |
| Talc | 1.0 |
| Magnesium stearate | 2.0 |
| | 200.0 |

### Example 3

| Tablets | |
|---|---|
| mg/tablet | |
| TZE-5323 | 100.0 |
| Starch | 10.0 |
| Lactose | 116.0 |
| Carboxymethylcellulose calcium | 20.0 |
| Talc | 1.5 |
| Magnesium stearate | 2.5 |
| | 250.0 |

## Claims

1. A pharmaceutical composition for the treatment or prevention of endometriosis and/or abnormal menstruation which contains an estrogen antagonist as an active ingredient, the pharmaceutical composition being **characterized in that** it is administered for only 5 to 13 consecutive days during a period of time extending from the menstrual period to immediately before ovulation in the menstrual cycle.

2. A pharmaceutical composition as claimed in claim 1 wherein the period of administration comprises only 7 to 9 consecutive days during a period of time extending from the menstrual period to immediately before ovulation in the menstrual cycle.

3. A pharmaceutical composition as claimed in claim 1 wherein the period of administration is determined so as to begin on the 3rd to 5th day after the onset of menstruation in the menstrual cycle and terminate on the 9th to 11th day after the onset of menstruation.

4. A pharmaceutical composition as claimed in claim 1 wherein the abnormal menstruation is dysmenorrhea, hypermenorrhea, hypomenorrhea, oligomenorrhea, polymenorrhea or paramenia.

5. A pharmaceutical composition as claimed in claim 1 wherein the estrogen antagonist is tamoxifen, raloxifene, clomiphene, toremifene, droloxifene or (6-hydroxy-2-cyclohexylbenzo[b]thien-3-yl) [4-[2-(1-piperidinyl)ethoxy]phenyl]methanone.

6. An agent for the treatment or prevention of endometriosis and/or abnormal menstruation which contains an estrogen antagonist as an active ingredient, the agent being administered for only 5 to 13 consecutive days during a period of time extending from the menstrual period to immediately before ovulation in the menstrual cycle.

7. A method for the treatment or prevention of endometriosis and/or abnormal menstruation which comprises administering an estrogen antagonist for only 5 to 13 consecutive days during a period of time extending from the menstrual period to immediately before ovulation in the menstrual cycle.

8. The use of an estrogen antagonist in the preparation of a pharmaceutical composition for the treatment or prevention of endometriosis and/or abnormal menstruation which is administered for only 5 to 13 consecutive days during a period of time extending from the menstrual period to immediately before ovulation in the menstrual cycle.

9. A pharmaceutical product for the treatment or prevention of endometriosis which includes a packaging material and an estrogen antagonist-containing pharmaceutical preparation within the packaging material, the pharmaceutical product being **characterized by** having, on or within the packaging material, an indication or document showing the instruction that the estrogen antagonist-containing pharmaceutical preparation should be administered for only 5 to 13 consecutive days during a period of time extending from the menstrual period to immediately before ovulation in the menstrual cycle.
